# FASCICULE DE BREVET EUROPEEN

(11) **EP 2 055 225 B1**
(45) Date de publication et mention de la délivrance du brevet: **05.12.2012**
(21) Numéro de dépôt: 08167785.8
(22) Date de dépôt: 29.10.2008
(51) Int. Cl.: A61B 1/00, A61B 1/31, A61B 17/34

(54) **Trocart endorectal**
Endorektaler Trokar (Punktionsnadel)
Endorectal trocar

(30) Priorité: 31.10.2007 FR 0758738
(43) Date de publication de la demande: 06.05.2009
(73) Titulaire: Aspide Medical, 42350 La Talaudiere (FR); Cohen, Jean-Pierre, 06250 Mougins (FR)
(72) Inventeur: Cohen, Jean-Pierre, 06250 Mougins (FR)
(74) Mandataire: Dupuis, François

(56) Documents cités:
- EP-A- 0 873 721
- EP-A- 1 219 251
- WO-A-02/17800
- FR-A- 2 710 270
- FR-A- 2 904 210
- US-A- 5 957 947

## Description

La présente invention concerne un trocart endorectal. En particulier, elle s'applique aux domaines de l'endocospie et de la chirurgie pour des interventions par voie rectale, pour des actes exploratoires ou thérapeutiques. Dans ce cadre, le terme trocart doit s'entendre de façon large comme un accessoire apte à introduire des instruments divers (en particulier d'endoscopie) au travers de cavités naturelles et, dans le cas de l'invention, dans le rectum par l'anus.

Dans ce domaine, des dispositifs très spécifiques sont généralement employés.

On connaît du document FR 2 536 651, un ensemble complexe spécifiquement destiné à la rectoscopie incluant un corps métallique apte à être introduit par voie rectale et disposant de son volume intérieur d'une pluralité d'instruments d'endoscopie du type caméra vidéo, système de gonflage endorectal ou autre. Ce dispositif complexe et coûteux comporte de multiples pièces.

De par sa conception, il est systématiquement réutilisable et nécessite des étapes délicates et coûteuses de stérilisation. Par ailleurs, l'étanchéité autour des voies de passage des instruments est garantie par une bague rapportée par l'extérieur du trocart et donc située en arrière du canal d'introduction au travers du rectum. Cette situation offre peu de débattement aux instruments introduits et met en oeuvre des moyens d'assemblage coûteux entre le corps de l'instrument et la bague d'étanchéité.

Il existe donc un besoin de proposer un trocart endorectal présentant une coopération plus efficace entre les moyens d'étanchéité interne du canal d'introduction des instruments et le corps du dispositif de trocart. Il existe aussi un besoin d'améliorer les étapes de mise en place du trocart notamment en ce qui concerne la phase d'introduction par l'anus et les étapes d'utilisation générale du trocart en particulier pour la réalisation de sa fixation, l'insufflation d'un fluide de gonflage etc.

On connaît par le brevet EP 1 219 251 un trocart conforme au préambule de la revendication 1, avec une membrane en position proximale et non amovible

La présente invention s'inscrit dans ce cadre et propose à cet effet un trocart endorectal amélioré. Selon l'invention, le trocart, avantageusement à usage unique, comporte un corps extérieur délimitant un canal interne permettant le passage des instruments ou encore d'un organe de dilatation préalable facilitant l'introduction. Au niveau de la paroi intérieure du corps, des moyens de fixation sont prévus au coeur même du canal interne pour coopérer avec une barrière étanche rapportable à l'intérieur du corps ou, avantageusement, avec un organe de dilatation.

Le placement de la barrière étanche au coeur même du canal interne permet de profiter pleinement d'une grande capacité de débattement des instruments et assure un plus grand volume de travail pour le praticien au niveau de l'extrémité extérieure du trocart. Les moyens de fixation utilisés selon l'invention sont prévus à cet effet dans le canal interne et comportent avantageusement des moyens de fixation rapide de sorte à permettre au praticien dans un premier temps de faire coopérer le corps avec un organe de dilatation pour l'introduction puis de retirer ledit organe pour le remplacer par la barrière étanche assurant le passage étanche des instruments d'endoscopie.

Etant au coeur du canal interne du corps, la barrière étanche peut y présenter un diamètre sensiblement équivalent et peut autoriser le passage simultané d'instruments multiples au travers de passages, sans blocage, rétrécissement ou désaxage entre le passage et le canal interne.

Toutes ces opérations s'effectuent très facilement de par la conception du trocart en particulier dans le cas préféré où un outil sous forme de poignée est utilisé pour la mise en place de la barrière étanche.

La conception utilisée et notamment celle de la barrière étanche assure en outre à la fois une conception peu coûteuse et une étanchéité de grande efficacité, de sorte que l'ensemble constitue non seulement un progrès technique mais un gain en matière de coût évitant les utilisations multiples de pièces et les stérilisations associées.

D'autres buts et avantages apparaîtront au cours de la description qui suit qui présente un mode préféré de réalisation de l'invention cependant non limitatif.

Auparavant, il est rappelé que la présente invention concerne un trocart endorectal pourvu d'un corps d'introduction endorectale délimitant un canal interne débouchant, d'une barrière étanche s'étendant transversalement au canal interne et présentant des passages pour instruments de rectoscopie, caractérisé par le fait que le corps et la barrière étanche sont deux ensembles distincts assemblables par des moyens de fixation au niveau du canal interne

Suivant des variantes données ci-après à titre non limitatif mais préféré le trocart est tel que :
- il comporte des moyens de manipulation de la barrière étanche depuis l'extérieur du canal interne,
- les moyens de manipulation comportent des poignées dont l'extrémité distale coopèrent de façon amovible avec la barrière étanche,
- l'extrémité distale de la poignée comporte au moins deux branches applicables sur une surface d'entraînement de la barrière étanche,
- les branches sont déformables élastiquement entre une position d'application sur la surface d'entraînement et une position libre,
- la poignée comporte un manche avec une zone déformable élastiquement configurée pour provoquer la déformation élastique des branches,
- la zone élastique déformable élastiquement comporte un évidement orienté suivant la direction longitudinale de la poignée et formé de sorte à délimiter une bordure déformable liée aux branches,
- les moyens de fixation comportent un système mâle/femelle de verrouillage par rotation,
- le dispositif comporte un organe de dilatation présentant une extrémité distale s'évasant progressivement et apte à coopérer avec le canal interne en l'absence de la barrière étanche,
- la surface extérieure de l'organe de dilatation rejoint la bordure distale du corps,
- l'extrémité distale de l'organe de dilatation présente une surface extérieure bombée,
- le corps et l'organe de dilatation sont assemblables par les moyens de fixation au niveau du canal interne.
- la barrière étanche comporte une pièce d'étanchéité intercalée entre deux flancs assemblés
- le dispositif comporte un chenal longitudinal dans l'épaisseur de la paroi du corps recevant un conduit d'arrivée d'un fluide de gonflage
- l'embouchure du chenal vers l'extrémité distale du trocart a une composante transversale,
- la barrière étanche est située sensiblement à la moitié de la longueur du canal interne.

Les dessins ci-joints sont donnés à titre d'exemples et ne sont pas limitatifs de l'invention. Ils représentent seulement un mode de réalisation de l'invention et permettront de la comprendre aisément.
Les figures 1 à 3 représentent un mode de réalisation du corps du trocart respectivement vu de côté, en coupe longitudinale et en vue de dessus.

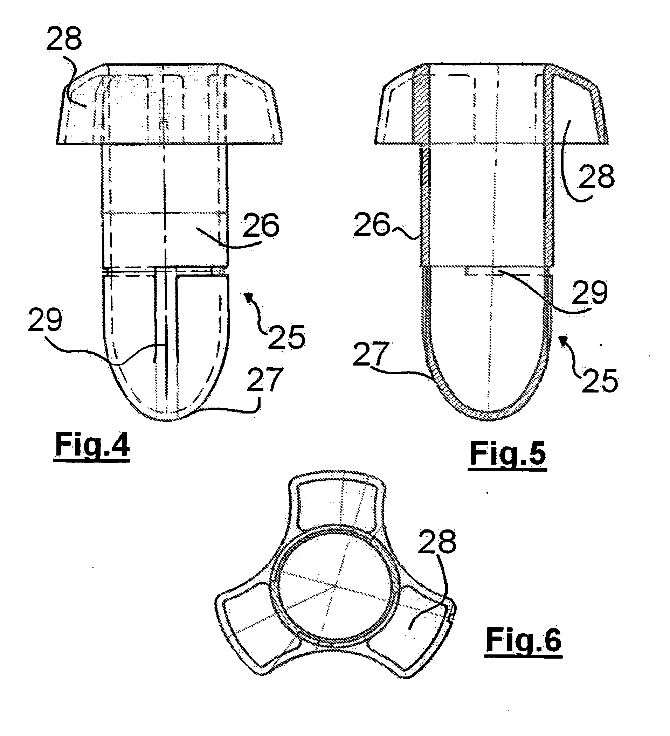

Les figures 4 à 6 présentent un mode de réalisation de l'organe de dilatation respectivement en vue de côté, en coupe longitudinale et en vue en coupe transversale.
Les figures 7 à 9 montrent de façon similaire la coopération entre le corps et l'organe de dilatation.
Les figures 10 et 11 montrent une pièce composant la barrière étanche et les figures 12 à 13 une deuxième pièce.
Les figures 14 à 16 illustrent la coopération entre le corps et la barrière étanche respectivement en vue de côté, en coupe longitudinale et en vue de dessus.
La figure 17 est un agrandissement de la figure 15.
La figure 18 présente de façon isolée un mode de réalisation de la poignée.

Le trocart ici décrit avantageusement destiné à l'endoscopie est préférentiellement conçu de manière à être jetable après un usage unique. A cet effet, les matériaux utilisés sont prévus en conséquence. Notamment, la majorité des pièces hormis la pièce d'étanchéité 3 qui sera décrite plus loin pourront être réalisées en matière plastique rigide moulée. La pièce d'étanchéité 3 peut, elle, être en polymère élastomère ou en silicone.

Par ailleurs, par convention, pour le reste de la description, on entend par "distale" l'extrémité des pièces du trocart situées vers l'intérieur du rectum et par laquelle débute l'introduction anale. On entend par "proximale" l'extrémité opposée à savoir celle qui reste accessible par le praticien une fois la mise en place du trocart effectué.

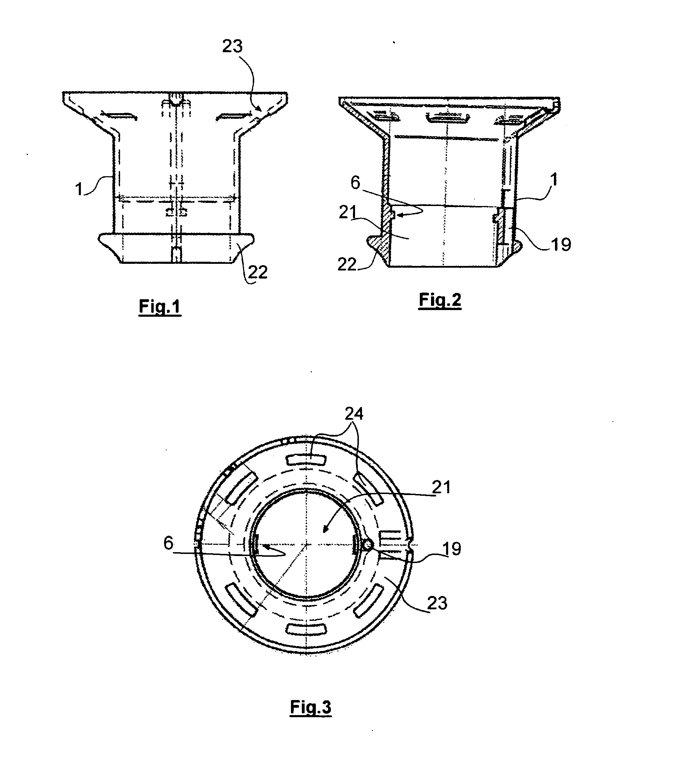

Le corps 1 illustré aux figures 1 à 3 comporte une paroi extérieure permettant l'introduction par voie rectale par l'intermédiaire d'une première extrémité ici dénommée bordure distale 22 dont la surface extérieure est évasée de sorte à faciliter l'introduction et présentant avantageusement un bourrelet relativement au reste du corps 1 de sorte à accentuer la rétention du trocart au niveau du bord supérieur du canal anal. La bordure proximale 23 du corps 1 reste accessible pour le praticien, notamment pour opérer l'introduction des instruments d'endoscopie. Cette extrémité est avantageusement évasée de sorte à offrir plus de volume de travail à l'opérateur, ce qui permet de dégager sur le côté l'arrivée du conduit d'insufflation de fluide de gonflage de sorte à éviter toute interférence avec les gestes du praticien.

Entre ces deux bordures 22, 23, le corps 1 comporte une portion intermédiaire dont la surface extérieure est préférentiellement cylindrique de même que la paroi interne délimitant un canal interne 21 assurant le passage d'instruments divers dont instruments d'endoscopie ou organe de dilatation 25. Le canal interne 21 est avantageusement de section cylindrique continue le long du trocart sans rétrécissement de sorte à ne pas gêner le passage simultané de plusieurs instruments. Une pluralité de lumières 24 formées sur la partie évasée de la bordure proximale 23 permettent une liaison du trocart avec le corps du patient par suture.

La paroi interne du corps 1 comporte par ailleurs des moyens de fixation 6 dont la fonction sera explicitée plus loin dans la description et pouvant comprendre deux saillies diamétralement opposées visibles aux figures 2 et 3 constituant des organes mâles susceptibles de coopérer avec des parois correspondantes sur d'autres pièces à rapporter sur le corps 1.

Les moyens de fixation 6 sont préférentiellement situés à l'intérieur du canal interne 21 sensiblement au niveau de la moitié de sa longueur pour un placement de la barrière étanche à ce niveau. D'une façon générale, la barrière étanche et les moyens de fixation sont avantageusement situés dans le tiers central de la longueur du canal interne.

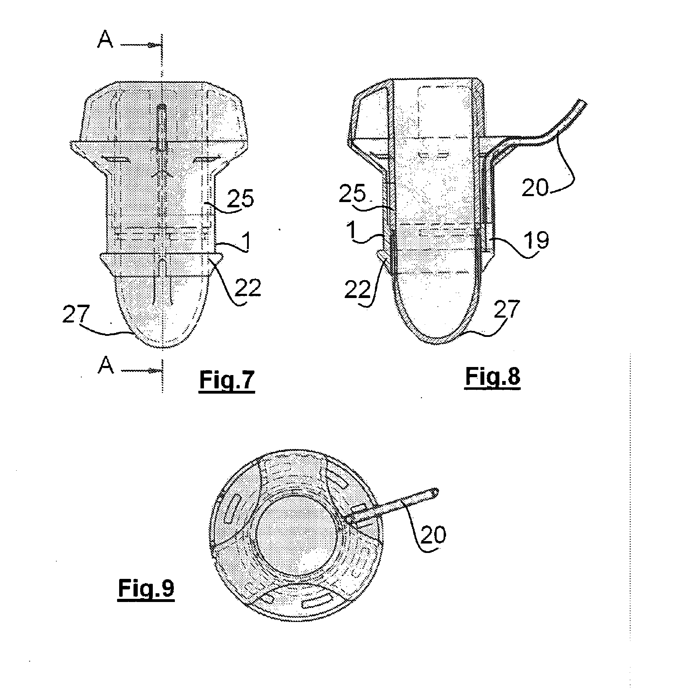

Le corps 1 comporte en outre avantageusement un chenal 19 orienté suivant sa direction longitudinale et s'étendant depuis la partie proximale jusqu'à la partie distale du corps 1 de sorte à recevoir un conduit 20 visible aux figures 8, 9, 15 et 16 pour l'amenée du fluide de gonflage. Cette solution simplifie la conception de la fabrication du corps 1 en particulier pour son moule d'injection.

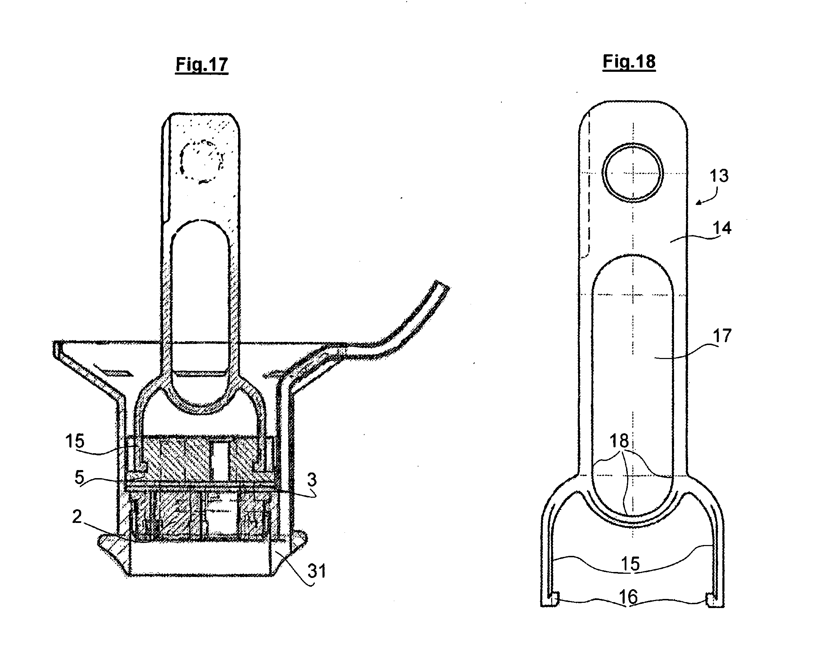

Le chenal 19 est par exemple une rainure moulée dans le corps 1 ou usinée dans laquelle est monté le conduit 20 qui peut alors être encastré notamment par un simple collage dans la rainure. Au niveau de sa partie distale, le chenal 19 comporte une embouchure apparaissant plus précisément en figure 17 sous la référence 31 de sorte à réaliser la sortie de gaz de gonflage dans le corps humain.

Dans le cas représenté, l'embouchure 31 comporte une sortie longitudinale et à la fois transversale pour le fluide. La composante transversale est avantageuse pour éviter l'obstruction intempestive du circuit de gonflage, qui pourrait être engendrée notamment par les muqueuses. Dans le cas illustré, par conséquent, une partie du gaz de gonflage est orientée vers l'embouchure distale du canal interne 21.

Les possibilités offertes par le trocart en ce qui concerne l'ergonomie pour le praticien ou les possibilités d'introduction d'instruments sont d'autant plus importantes que le diamètre du corps 1 est grand. Pour utiliser un trocart suffisamment large, le corps 1 coopère avantageusement selon l'invention avec un organe de dilatation 25 comportant une zone longitudinale 26 sensiblement allongée et qui peut être introduite au travers du canal interne 21 du corps 1. L'organe de dilatation 25 comporte en outre une extrémité distale 27 de section circulaire progressive et une zone de préhension 28 située à l'opposé de l'extrémité distale 27 de sorte à faciliter les manipulations du praticien. La partie 28 s'applique avantageusement sur le pourtour de la bordure 23 du corps 1 comme cela est visible aux figures 7 à 9.

Une échancrure 29 formée sur la surface extérieure de la zone longitudinale 26 de l'organe de dilatation 25 de façon correspondante avec les moyens de fixation 6 de la paroi interne du corps 1 permettent leur coopération. En particulier, une translation longitudinale de l'organe de dilatation 25 dans le canal interne 21 est suivie d'une rotation d'un angle limité de sorte à faire passer successivement les moyens de fixation 6 d'une coopération avec une portion longitudinale de l'échancrure 29 à une portion radiale de cette échancrure.

Une fois mis en place et assemblé, l'organe de dilatation 25 assure une introduction facilitée au travers de la cavité naturelle du patient. L'aisance est d'autant plus grande que l'extrémité distale 27 de l'organe de dilatation 25 a une forme choisie pour s'inscrire dans la continuité de la surface extérieure de la bordure distale 22 du corps 1. Cette continuité est particulièrement visible aux figures 7 et 8. On comprend aisément que l'introduction est parfaitement progressive sur la partie bombée de l'extrémité distale de l'organe de dilatation 25 jusqu'à atteindre la bordure 22 et son bourrelet de rétention apte à fixer en position le corps 1 relativement à la cavité naturelle.

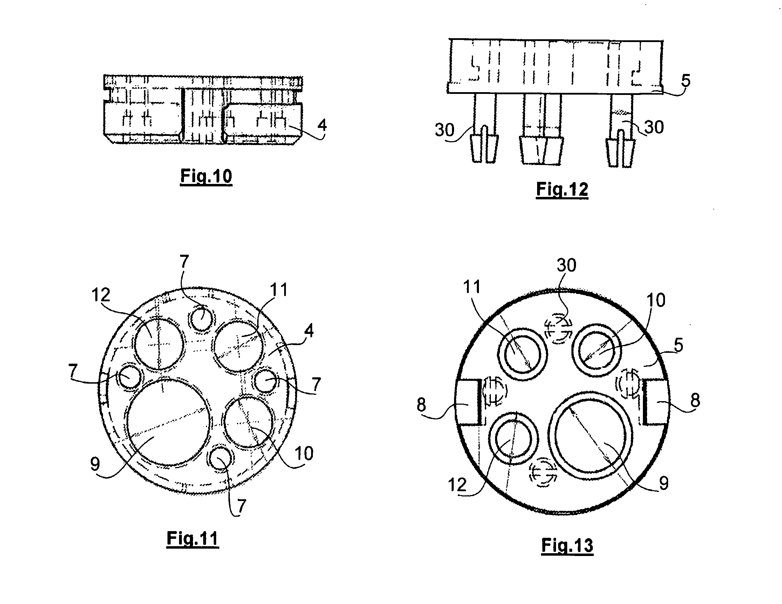

Les figures 10 à 13 présentent un autre aspect du trocart par la conception spécifique de la barrière étanche 2. Cette barrière 2 a pour fonction d'assurer l'étanchéité périphérique avec le corps 1 et l'étanchéité au niveau de chacun des passages 9, 10, 11, 12 permettant l'introduction d'instruments d'endoscopie. Le nombre de passages 9, 10, 11, 12 n'est pas limité ni même leur diamètre ou leur configuration. On a représenté à titre indicatif la formation de trois passages 10, 11, 12 d'un premier diamètre et la réalisation d'un passage 9 de plus grand diamètre pouvant s'adapter à différents types d'instruments.

Dans le cas illustré, la barrière étanche 2 est formée en plusieurs pièces à savoir un flanc distal 4 coopérant avec un flanc proximal 5 de sorte à encadrer une pièce d'étanchéité 3 prise en étau. Les moyens d'assemblage entre les deux flancs 4, 5 ne sont pas limités et on a représenté à titre préféré la réalisation d'une pluralité de tiges d'assemblage 30 faisant saillie sur la surface intérieure du flanc proximal 5 et pouvant être introduites dans des trous d'assemblage 7 formés en correspondance au travers du flanc distal 4. Une extrémité échancrée avec une portion conique dotée d'un rebord au niveau des tiges 30 assure un verrouillage en fin d'introduction des tiges 30 dans les trous d'assemblage 7. Cet assemblage s'effectue après une mise en place de façon intercalaire d'une pièce d'étanchéité 3 en matériau souple tel un élastomère. L'ensemble des flancs 4, 5 et de la pièce d'étanchéité 3 sont évidemment formés de sorte à présenter des trous en correspondance pour constituer chacun des passages 9, 10, 11, 12. La pièce d'étanchéité 3 est avantageusement d'un diamètre légèrement plus grand pour s'appliquer sur la paroi intérieure du corps 1. De même, son diamètre au niveau de chaque passage 9, 10, 11, 12 est avantageusement légèrement plus petit de sorte à constituer un contact étanche autour des instruments à introduire.

On comprend aisément que cette conception de barrière étanche 2 assure à la fois un maintien en position efficace d'une pièce d'étanchéité 3 qui peut être choisie selon des considérations de capacité d'étanchéisation alors que les flancs 4 et 5 sont dévolus à la rigidification et à la résistance mécanique de la barrière. Une étanchéité parfaite est donc obtenue sans pour autant impacter la résistance mécanique de la barrière étanche.

De façon similaire à la coopération entre le corps 1 et l'organe de dilatation 25, la barrière étanche 2 dispose de moyens susceptibles de coopérer avec les moyens de fixation 6. Ainsi, une échancrure 29 disposant d'une portion longitudinale et d'une portion radiale est constituée sur le flanc distal 4 pour l'introduction des moyens de fixation 6 et un verrouillage par rotation.

Cette configuration est bien entendu uniquement indicative et non limitative.

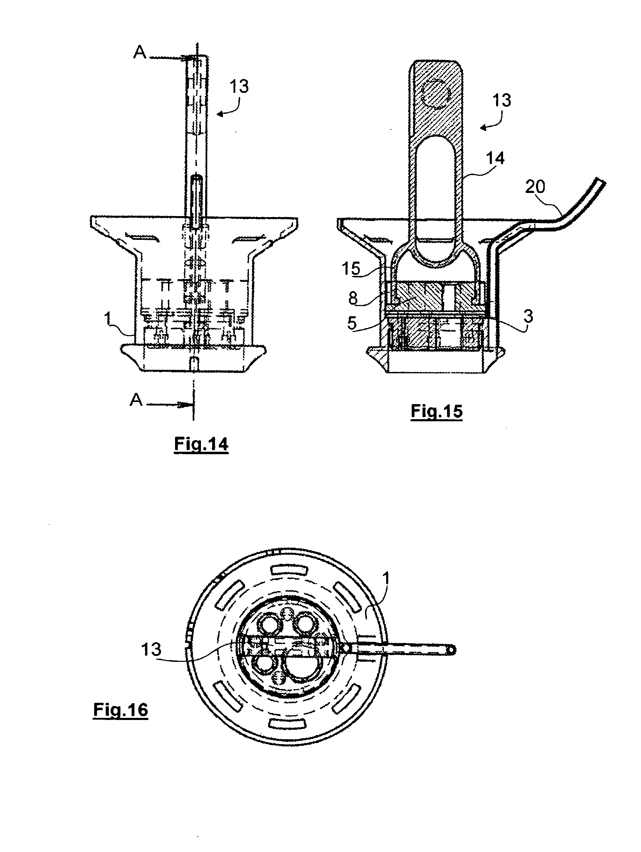

Du fait de la présence de la barrière étanche 2 au milieu du canal 21, il est préférable d'opérer sa mise en place par l'intermédiaire d'un outil ad hoc pouvant être constitué par une poignée 13 apparaissant en détail en figure 18 et dont la coopération avec la barrière étanche 2 est illustrée aux figures 14 à 17.

Cette poignée 13 constitue des moyens d'actionnement à distance de la barrière étanche 2 permet d'en déporter la manipulation vers l'extrémité proximale du trocart. Des alternatives à la poignée 13 ci-dessous explicitée sont dans le champ de l'invention. En particulier, la barrière étanche 2 peut être solidaire d'un manchon cylindrique creux de faible épaisseur et s'appliquant sur la paroi interne du corps 1 entre la barrière étanche 20 et la bordure proximale 23 de sorte à constituer un organe d'actionnement déporté de la barrière étanche.

Dans le cas illustré, la poignée 13 comporte un manche 14 dont une partie dispose d'un évidement 17 ici sous forme de trou oblong orienté suivant l'axe longitudinal de la poignée 13 et permettant de délimiter une bordure 18 déformable élastiquement en particulier par compression entre deux doigts du praticien. La bordure 18 est elle-même en liaison avec des branches 15 au nombre de deux dans le cas illustré. L'extrémité distale des branches 15 comporte un rebord 16 apte à coopérer avec une portion correspondante dans des créneaux 8 formés dans le flanc proximal 5 de la barrière étanche 2. Les créneaux 8 forment une surface d'entraînement pour la mobilité de la barrière étanche 2 en fonction de sa coopération avec la poignée 13.

Avantageusement, les branches 15 sont déformables élastiquement de sorte à passer d'une position de verrouillage relativement aux créneaux 8 à une position libre pour leur mise en place ou leur extraction. A cet effet, on profite d'une déformation élastique de la bordure 18 pour induire un écartement ou un rapprochement des branches 15. Plus précisément, une pression du praticien sur les bords parallèles longitudinaux de la bordure 18 provoque leur rapprochement et la déformation de la partie intermédiaire de la bordure 18 induit une rotation des branches 15 qui ont tendance à s'écarter. A l'inverse, un relâchement de la pression de la part du praticien permet un retour en position de repos de l'ensemble.

La mise en place de la barrière étanche 2 dans le corps 1 s'effectue donc de façon déportée particulièrement facile pour le praticien. En effet, une fois la barrière étanche 2 assemblée avec la poignée 13, le praticien n'a plus qu'à en opérer l'introduction dans le canal interne 21 et ce sans aucune interférence avec la paroi interne du corps 1. Une fois la mise en place opérée, il suffit d'une rotation d'un angle limité (par exemple de l'ordre de 30°) de la barrière étanche 2 pour la fixer relativement au corps 1.

L'introduction des instruments d'endoscopie peut alors être opérée.

On notera que la barrière étanche 2 constituant une pièce rapportée et amovible peut être facilement remplacée en cas de problème d'étanchéité ou si sa configuration (nombre de trous, diamètre) ne devait plus convenir au geste exploratoire ou chirurgical que le praticien souhaite entreprendre. Cela étant, on peut aussi prévoir une fixation définitive garantissant contre des incidents en phase opératoire.

### REFERENCES

- 1.: Corps
- 2.: Barrière étanche
- 3.: Pièce d'étanchéité
- 4.: Flanc distal
- 5.: Flanc proximal
- 6.: Moyens de fixation
- 7.: Trous d'assemblage
- 8.: Créneau
- 9.: Passage
- 10.: Passage
- 11.: Passage
- 12.: Passage
- 13.: Poignée
- 14.: Manche
- 15.: Branche
- 16.: Rebord
- 17.: Evidement
- 18.: Bordure déformable
- 19.: Chenal
- 20.: Conduit
- 21.: Canal interne
- 22.: Bordure distale
- 23.: Bordure proximale
- 24.: Lumière de suture
- 25.: Organe de dilatation
- 26.: Zone longitudinale
- 27.: Extrémité distale
- 28.: Zone de préhension
- 29.: Echancrure
- 30.: Tige d'assemblage
- 31.: Embouchure

## Revendications

1. Trocart endorectal pourvu :
a- d'un corps (1) d'introduction endorectale délimitant un canal interne (21) débouchant,
b- d'une barrière étanche (2) s'étendant transversalement au canal interne (21) et présentant des passages (9, 10, 11, 12) pour instruments de rectoscopie, le corps (1) et la barrière étanche (2) étant deux ensembles distincts assemblables par des moyens de fixation (6) au niveau du canal interne (21)
**caractérisé en ce que** la barrière étanche est disposée sensiblement à la moitié de la longueur du canal interne, et **en ce qu'**il comprend des moyens de manipulations de la barrière étanche (2) depuis l'extérieur du dit canal interne (21), et **en ce que** les dits moyens de manipulation comportent des poignées (13) dont l'extrémité distale coopèrent de façon amovible avec la barrière étanche (2), et **en ce que** la barrière étanche (2) comprend un flanc distal (4) coopérant avec un flanc proximal (5) entre lesquels est disposée une pièce d'étanchéité (3) prise en étau, et **en ce que** la barrière étanche (2) est aménagée avec des moyens d'entraînement (8) pour coopérer avec la poignée (1), les dits moyens (8) étant des créneaux formés sur le flanc proximal (5) de la dite barrière, et **en ce que** le corps (1) coopère avec un organe de dilatation (25) agencé avec une échancrure (29) disposée en regard des dits moyens de fixation (6), et **en ce que** les dits moyens de manipulation sous forme de poignée (13) présentent des branches (15) pour coopérer avec la dite surface d'entraînement (8) de la barrière étanche, et **en ce que** la mise en place ou enlèvement de la barrière étanche s'effectue par un opération complémentaire de rotation de la barrière étanche par rapport au dit corps (1).

2. Trocart selon la revendication 1 dans lequel l'extrémité distale de la poignée (13) comporte au moins deux branches (15) applicables sur une surface d'entraînement de la barrière étanche (2).

3. Trocart selon la revendication 2 dans lequel les branches (15) sont déformables élastiquement entre une position d'application sur la surface d'entraînement et une position libre.

4. Trocart selon la revendication 3 dans lequel la poignée (13) comporte un manche (14) avec une zone déformable élastiquement configurée pour provoquer la déformation élastique des branches (15).

5. Trocart selon la revendication 4 dans lequel la zone déformable élastiquement comporte un évidement (17) orienté suivant la direction longitudinale de la poignée (13) et formé de sorte à délimiter une bordure déformable (18) liée aux branches (15).

6. Trocart selon une quelconque des revendications 1 à 5 comportant un organe de dilatation (25) présentant une extrémité distale s'évasant progressivement et apte à coopérer avec le canal interne (21) en l'absence de la barrière étanche (2).

7. Trocart selon la revendication 6 dans lequel la surface extérieure de l'organe de dilatation (25) rejoint la bordure distale (22) du corps (1).

8. Trocart selon la revendication 6 ou 7 dans lequel l'extrémité distale de l'organe de dilatation présente une surface extérieure bombée.

9. Trocart selon la revendication 6 ou 7 ou 8 dans lequel le corps (1) et l'organe de dilatation (25) sont assemblables par les moyens de fixation au niveau du canal interne (21).

10. Trocart selon la revendication 1 comportant un chenal (19) longitudinal dans l'épaisseur de la paroi du corps (1) recevant un conduit (20) d'arrivée d'un fluide de gonflage.

11. Trocart selon la revendication 10 dans lequel l'embouchure du chenal (19) vers l'extrémité distale du trocart a une composante transversale.

## Claims

1. Endorectal trocar provided with:
a - a body (1) for endorectal introduction that defines an open internal channel (21)
b - a leak-tight barrier (2) that extends across the internal channel (21) and has passageways (9, 10, 11, 12) for rectoscopy instruments, the body (1) and the leak-tight barrier (2) being two distinct assemblies that can be attached by fixing means (6) at the internal channel (21)
**characterised in that** the leak-tight barrier is arranged substantially at mid length of the internal channel, and **in that** it comprises means of handling the leak-tight barrier (2) from outside said internal channel (21), and **in that** said handling means comprise handles (13) the distal end of which cooperates removably with the leak-tight barrier (2), and **in that** the leak-tight barrier (2) comprises a distal side (4) cooperating with a proximal side (5) between which a sealing part (3) is gripped, and **in that** the leak-tight barrier (2) is provided with coupling means (8) to cooperate with the handle (13), the said means (8) being slots formed on the proximal side (5) of the said barrier, and **in that** the body (1) cooperates with a dilation device (25) provided with a notch (29) arranged opposite said fixing means (6), and **in that** said handling means in the form of a handle (13) have arms (15) to cooperate with the said coupling face (8) of the leak-tight barrier, and **in that** the positioning or removal of the leak-tight barrier is achieved by an additional rotation operation of the leak-tight barrier in relation to the body (1).

2. Trocar according to claim I wherein the distal end of the handle (13) comprises at least two arms (15) that can be applied to a coupling face of the leak-tight barrier (2).

3. Trocar according to claim 2 wherein the arms (15) are elastically deformable between a position in which they are applied to the coupling face and a free position.

4. Trocar according to claim 3 wherein the handle (13) comprises a sleeve (14) with an elastically defonnable zone configured to cause the elastic deformation of the arms (15).

5. Trocar according to claim 4 wherein the elastically deformable zone comprises a recess (17) oriented along the longitudinal direction of the handle (13) and formed so as to define a deformable border (18) linked to the arms (15).

6. Trocar according to any of claims I to 5 comprising a dilation device (25) with a distal end gradually flaring out and capable of cooperating with the internal channel (21) in the absence of the leak-tight barrier (2).

7. Trocar according to claim 6 wherein the outer surface of the dilation device (25) meets with the distal edge (22) of the body (1).

8. Trocar according to claim 6 or 7, wherein the distal end of the dilation device has a rounded outer surface.

9. Trocar according to claim 6 or 7 or 8 wherein the body (1) and the dilation device (25) can be attached by fixing means at the internal channel (21).

10. Trocar according to claim 1 comprising a longitudinal channel (19) within the wall of body (1) receiving an inlet tube (20) for an inflation fluid.

11. Trocar according to claim 10 wherein the opening of the channel (19) to the distal end of the trocar has a transverse component.

## Patentansprüche

1. Endorektaltrokar mit:
a - einem Körper (1) für die endorektale Einführung, der einen ausführenden internen Kanal (21) umgrenzt,
b - einer dichten Barriere (2), die sich quer über den internen Kanal (21) erstreckt und Durchlässe (9, 10, 11, 12) für Rektoskopieinstrumente aufweist,
wobei der Körper (1) und die dichte Barriere (2) zwei unterschiedliche Einheiten sind, die durch Befestigungseinrichtungen (6) im Bereich des internen Kanals (21) verbunden werden können,
**dadurch gekennzeichnet, dass** die dichte Barriere ziemlich genau in der Mitte der Länge des internen Kanals angeordnet ist, und dass der Trokar Einrichtungen für die Handhabung der dichten Barriere (2) von der Außenweite des besagten internen Kanals (21) her umfasst, und dass die besagten Handhabungseinrichtungen Griffe (13) aufweisen, deren distales Ende abnehmbar mit der dichten Barriere (2) zusammenwirkt, und dass die dichte Barriere (2) eine distale Flanke (4) umfasst, die mit einer proximalen Flanke (5) zusammenwirkt, zwischen denen ein Dichtigkeitsstück (3) eingezwängt ist, und dass die dichte Barriere (2) mit Mitnahmeeinrichtungen (8) ausgestattet ist, um mit dem Griff (13) zusammenzuwirken, wobei die besagten Einrichtungen (8) an der proximalen Flanke (5) der besagten Barriere ausgebildete Zacken sind, und dass der Körper (1) mit einem Dehnungsorgan (25) zusammenwirkt, der mit einem Ausschnitt (29) versehen ist, der den besagten Befestigungseinrichtungen (6) zugewandt ist, und dass die besagten Handhabungseinrichtungen in Form eines Griffs (13) Bügel (15) aufweisen, um mit der besagten Mitnahmefläche (8) der dichten Barriere zusammenzuwirken, und dass das Einsetzen bzw. Entfernen der dichten Barriere durch eine zusätzlichen Drehbewegung der dichten Barriere gegenüber dem besagten Körper (1) erfolgt.

2. Trokar nach Anspruch 1, bei dem das distale Ende des Griffs (13) mindestens zwei Bügel (15) umfasst, die auf eine Mitnahmefläche der dichten Barriere (2) einsetzbar sind.

3. Trokar nach Anspruch 2, bei dem die Bügel (15) zwischen einer Einsetzposition auf der Mitnahmefläche und einer freien Position elastisch verformbar sind.

4. Trokar nach Anspruch 3, bei dem der Griff (13) einen Stiel (14) mit einem elastisch verformbaren Bereich umfasst, der so ausgelegt ist, um die elastische Verformung der Bügel (15) zu bewirken.

5. Trokar nach Anspruch 4, bei dem der elastisch verformbare Bereich eine Aussparung (17) umfasst, die in der Längsrichtung des Griffs (13) ausgerichtet und so ausgebildet ist, um eine mit den Bügeln (15) verbundene verformbare Kante (18) zu umgrenzen.

6. Trokar nach einem der Ansprüche 1 - 5 mit einem Dehnungsorgan (25) mit einem distalen Ende, das sich zunehmend ausweitet und geeignet ist, in Abwesenheit der dichten Barriere (2) mit dem internen Kanal (21) zusammenzuwirken,

7. Trokar nach Anspruch 6, bei dem die Außenfläche des Dehnungsorgans (25) mit der distalen Kante (22) des Körpers (1) zusammentrifft.

8. Trokar nach Anspruch 6 oder 7, bei dem das distale Ende des Dehnungsorgans eine gewölbte Außenfläche aufweist.

9. Trokar nach Anspruch 6 oder 7 oder 8, bei dem der Körper (1) und das Dehnungsorgan (25) durch die Befestigungseinrichtungen im Bereich des internen Kanals (21) verbunden werden können.

10. Trokar nach Anspruch 1 mit einer länglichen Rinne (19) in der Materialdicke der Wand des Körpers (1), die eine Zuleitung (20) eines Aufblasmediums aufnimmt.

11. Trokar nach Anspruch 10, bei dem die Mündung der Rinne (19) zum distalen Ende des Trokars hin eine querverlaufende Komponente besitzt.
